# NEW EUROPEAN PATENT SPECIFICATION

(11) **EP 2 114 330 B2**
(45) Date of publication and mention of the opposition decision: **09.01.2019**
(45) Mention of the grant of the patent: 30.03.2016
(21) Application number: 08709960.2
(22) Date of filing: 07.02.2008
(51) Int. Cl.: A61F 13/15, A61L 15/56

(54) **DISPOSABLE ABSORBENT ARTICLES HAVING PHOTOCHROMIC INK BASED GRAPHICS**
SAUGFÄHIGE EINWEGARTIKEL MIT PHOTOCHROMEN GRAFIKEN AUF TINTENBASIS
ARTICLES ABSORBANTS JETABLES COMPRENANT DES GRAPHIQUES À BASE D'ENCRE PHOTOCHROME

(30) Priority: 08.02.2007 US 703947
(43) Date of publication of application: 11.11.2009
(73) Proprietor: The Procter & Gamble Company, Cincinnati, OH 45202 (US)
(72) Inventor: WARNER, Alrick, Vincent, Loveland, Ohio 45140-6210 (US)
(74) Representative: Heide, Ute
(86) International application number: PCT/IB2008/050451
(87) International publication number: WO 2008/096327

(56) References cited:
- EP-A- 1 591 131
- EP-A1- 1 591 131
- US-A- 4 987 849
- US-A- 5 581 090
- US-A- 5 766 389
- US-A- 6 132 681
- US-A1- 2001 053 898
- US-A1- 2005 133 401
- US-A1- 2006 020 249
- 'OPTICAL BRIGTENERS, FLUORESCENT WHITENING AGENTS FOR PLASTICS, PAINTS, IMAGING AND FIBERS' CIBA 1999, CIBA SPECIALTY CHEMICALS, BASEL, CH,

## Description

### FIELD OF THE INVENTION

The present invention relates to a disposable absorbent article comprising a graphic disposed on a surface of a backsheet wherein said graphic comprises a photochromic ink composition that becomes visible to the naked human eye upon exposure to ultraviolet light.

### BACKGROUND OF THE INVENTION

More often than not disposable absorbent articles are incorporating graphics as an ordinary feature. In most instances, these graphics are applied to the backsheet of the product so that the graphics are visible while the product is being worn. The graphics have been printed directly on a component of the backsheet or have been printed on a separate layer, such as a tissue layer, which is disposed on the backsheet. These graphics, however, may be disposed on other components of a diaper including, but not limited to, a topsheet that interfaces with the wearer of the diaper.

EP 1 591 131 A1 discloses absorbent articles of the present invention which are capable to perform colour changes in response to an external stimulus. These colour changes are visible to the users of such articles. The external stimuli herein are pressure, temperature and light.

There are many reasons to incorporate graphics in disposable absorbent articles. For instance, the graphics can improve the appearance and appeal of the product, to both the wearer and the purchaser. In the case of diapers, infants and caregivers of infants find that the graphics tend to make the diaper changing experience more fun and engaging. Graphics can also impact the manner in which a disposable absorbent article is used. For example, graphics on disposable diapers can be used by caregivers to amuse and/or educate a child during diapering. Similarly, graphics on disposable training pants can provide educational and motivational mechanisms to facilitate the toilet training process. Graphics on training pants can also serve to increase the child's interest in the product and thereby increase the child's interest in the toilet training process.

There arc a variety of graphic types that have been used in the disposable absorbent article setting. First, there are those graphics that are permanent. That is, these graphics remain uncharged on the article despite the environmental conditions. Next, there are disappearing graphics that become invisible when exposed to certain environmental changes like wetting. Then, there are appearing graphics that tend to reveal themselves when exposed to certain environmental changes like wetting. Applicants recognized that there is still a need to provide different types of appearing graphics. In particular, Applicants have found that the incorporation and/or application of a photochromic ink composition in the form of a graphic onto and/or into a component of an article is a desirable mechanism to deliver the appearing graphic. Such graphics would be quite useful in the field of diaper products that are typically exposed to direct sunlight. For instance, those diaper products that are worn as swimwcar possess an inherent utility since they are usually not covered by outer garments and are usually used outside in the sun.

Photochromic ink compositions have been used in a number of other fields over the years. The photochromic effect (photochromism) in general is a reversible change of a single chemical species between two states having distinguishably different absorption spectra, wherein the change is induced in at least one direction by the action of electromagnetic radiation. This inducing radiation, as well as the changes in the absorption spectra, is usually in the ultraviolet, visible or infrared regions. In some instances, the change in one direction may also be thermally induced. The single chemical species can be a molecule or an ion, and the reversible change in states may be a conversion between two molecules or ions, or the dissociation of a single molecule or ion into two or more species, with the reverse change being a recombination of the two or more species thus formed into the original molecule or ion. In general, photochromic phenomena are observed in both organic compounds, such as anils, disulfoxides, hydrazones, osazones, semicarbazones, stilbene derivatives, o-nitrobenzyl derivatives, spiro compounds, and the like, and in inorganic compounds, such as metal oxides, alkaline earth metal sulfides, titanates, mercury compounds, copper compounds, minerals, transition metal compounds such as carbonyls, and the like. Inks containing photochromic components have generally been used as a security ink, watermark or to create some other means for authenticating a document (e.g., a stock certificate). Applicants have surprisingly found that these same inks may be used to form images or graphics on a disposable absorbent article wherein the graphics become visible to the naked human eye when the graphic is exposed to ultraviolet light.

### SUMMARY OF THE INVENTION

The present invention, therefore, relates to disposable absorbent articles as defined in claim 1.

### BRIEF DESCRIPTION OF THE DRAWINGS

Figure 1 is a perspective view of an article made according to the present invention.
Figure 2 is a front plan view of the graphic depicted on the article of Figure 1 prior to being exposed to ultraviolet light.
Figure 3 is a front plan view of the graphic depicted on the article of Figure 1 after being exposed to ultraviolet light.

### DETAILED DESCRIPTION OF THE INVENTION

### DEFINITIONS

As used herein, the term "absorbent articles" refers to devices that absorb and contain body exudates, and, more specifically, refers to devices that are placed against or in proximity to the body of the wearer to absorb and contain the various exudates discharged from the body. Absorbent articles may include diapers, training pants, adult incontinence undergarments, feminine hygiene products, breast pads, care mats, bibs, wound dressing products, and the like. As used herein, the term "body fluids" or "body exudates" includes, but is not limited to, urine, blood, vaginal discharges, breast milk, sweat and fecal matter.

As used herein, the term "absorbent core" refers to the component of the absorbent article that is primarily responsible for fluid handling properties of the article, including acquiring, transporting, distributing and storing body fluids. As such, the absorbent core typically does not include the topsheet, backsheet or outer cover of the absorbent article.

As used herein, the term "bonded" refers to different materials being attached (cohesively or adhesively) in at least a portion thereof. The attached portions may be random or may have a pattern such as stripes, spirals, dots, and the like. The attached portions may be located at the peripheries, throughout the surface area, or both. Suitable attachment means known in the art may be used, including but not limited to adhesives, heat, pressure, crimping, ultrasonic, chemical (via hydrogen bonds or other cohesive forces), mechanical (e.g., fasteners, entanglements), hydraulic, vacuum and combinations thereof.

As used herein, the term "composite structure" refers to a multi-region structure wherein the materials comprising the regions may be operatively associated or bonded. The regions may even be in intimate contact such that the composite has a unitary structure. Further, the regions may be positioned in a layered (face-to-face) arrangement, or a side-by-side arrangement.

As used herein, the term "disposable" is used in its ordinary sense to mean an article that is disposed or discarded after a limited number of usage events over varying lengths of time, preferably less than about 20 events, more preferably less than about 10 events, even more preferably less than about 5 events, and most preferably less than about 2 events.

As used herein, the term "intimate bonding" refers to physical contact between two layers such that they resist separation with no readily visually identifiable areas of separation. In one particular embodiment, the inner and outer layers are adhesively laminated together in the graphic regions using a meltblowing process to form an overlapping network of adhesive filaments.

As used herein, the term "joined" encompasses configurations wherein an element is directly secured to the other element by affixing the element directly to the other element, and configurations wherein the element is indirectly secured to the other element by affixing the element to intermediate member(s), which in turn are affixed to the other element.

As used herein, the terms "nonwoven" and "nonwoven web" refers to a web that has a structure of individual fibers which are interlaid forming a matrix, but not in an identifiable repeating manner. Nonwoven webs may be formed by a variety of processes known to those skilled in the art, for example, meltblowing, spunbonding, wet-laying, air-laying, and various bonding-carding processes.

As used herein, the term "operatively associated" refers to a structure comprising different materials positioned at least in partial contact with each other in use. The materials are physically separable and each exhibits properties that can be measured individually. The materials may be arranged in a face-to-face relationship in the z-dimension, or in a side-by-side relationship in the xy-dimension.

As used herein, the term "photochromic" refers to an ink composition that is capable of changing its color appearance upon exposure to ultraviolet light. For instance, the ink composition may turn from being invisible to having a perceivable color when exposed to sunlight.

As used herein, the term "pulp" or "cellulosic fibers" include those natural fiber derived from trees or vegetations (e.g., hardwood fibers, softwood fibers, hemp, cotton, flax, esparto grass, milkweed, straw, bagasse and the like), their processed/regenerated fibers (e.g., Rayon®) or chemically derivatized fibers (e.g., cellulose esters), and combinations thereof. Suitable hardwood fibers include eucalyptus fibers. Suitable hardwood fibers may be prepared by kraft or other chemical pulping methods. Suitable softwood fibers include southern softwood (SS) fibers and northern softwood (NS) fibers. Softwood fibers for use herein can be chemically (e.g., without limitation, kraft pulp) or mechanically pulped (e.g., without limitation, chemithermal mechanical pulp (CTMP) and thermal mechanical pulp (TMP)).

As used herein, the term "region" refers to a zone or an area comprising a material being physically, chemically, or visually distinguishable from surrounding or adjoining materials. Various regions of materials may include transitional regions in between. The regions may be positioned in the z-dimension or in the xy-dimension. As used herein, the term "z-dimension" refers to the dimension orthogonal to the length and width of the structure or article. The z-dimension usually corresponds to the thickness of the structure or article. As used herein, the term "xy-dimension" refers to the plane orthogonal to the thickness of the member, core or article when the member, core or article is in a flat-out state. The xy-dimension usually corresponds to the length and width, respectively, of the structure or article in a flat-out state.

As used herein, the term "unitary structure" refers to a structure comprising materials having different characteristics joined together to form an integral entity such that the materials are substantially inseparable physically, and the unitary structure exhibits properties resulting from the combination of the materials therein. The materials may be arranged in a face-to-face relationship in the z-dimension, or in a side-by-side relationship in the xy-dimension. The following detailed description is directed toward absorbent articles. It is, however, likely that the disclosed articles could be modified such that the overall concept discussed herein could likewise be applied to use in other hygiene or health care products, such as bandages, dressings, wipes, bibs, surgical drapes, surgical gowns, and the like.

### ABSORBENT ARTICLE

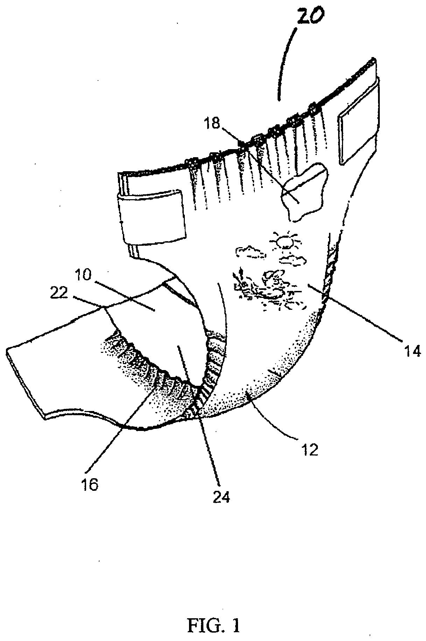

Figure 1 depicts the absorbent articles of the present invention, e.g., diaper(s) 20, which comprise a liquid pervious topsheet 10, a backsheet 12 that is at least partially joined to the topsheet 10, an absorbent core 18 disposed at least partially between the topsheet 10 and the backsheet 12, a first cuff 16 along a longitudinal edge 22 of the topsheet 10, and a graphic 14 printed on a garment-facing surface 24 (here, the surface is wearing facing) of said backsheet 12 wherein said graphic comprises a photochromic ink composition that becomes visible to the naked human eye upon exposure to ultraviolet light.

In certain embodiments, the absorbent articles may additionally include one or more components selected from the group consisting of an outer cover, side panels, an elastic feature, a fastening system, and combinations thereof.

An outer cover (which may comprise the backsheet) forms the chassis, onto which other components of the diaper are added to form the unitary structure of the diaper. In alternative embodiments, the article may be preformed by the manufacturer to create a pant. The term "pant", as used herein, refers to disposable garments having a waist opening and leg openings designed for infant or adult wearers. A pant may be placed in position on the wearer by inserting the wearer's legs into the leg openings and sliding the pant into position about the wearer's lower torso. A pant may be preformed by any suitable technique including, but not limited to, joining together portions of the article using refastenable and/or non-refastenable bonds (e.g., seam, weld, adhesive, cohesive bond, fastener, etc.). While the term "pant" is used herein, pants are also commonly referred to as "closed diapers", "prefastened diapers", "pull-on diapers", "training pants" and "diaper-pants". Suitable pants are disclosed in U.S. Patent Nos. 5,246,433; 5,569,234, 6,120,487, 6,120,489, 4,940,464, 5,092,861, 5,897,545, 5,957,908, and U.S. Patent Publication 2003/0233082A1.

### TOPSHEET

The absorbent articles of the present invention comprise a topsheet 10. The topsheet is preferably compliant, soft feeling, and non-irritating to the wearer's skin. It can be elastically stretchable in one or two directions. The topsheet has at least one longitudinal edge 22 and in most instances has two. Further, the topsheet is liquid pervious, permitting liquids (e.g., menses, urine, and/or runny feces) to readily penetrate through its thickness. A suitable topsheet can be manufactured from a wide range of materials such as woven and nonwoven materials; apertured or hydroformed thermoplastic films; porous foams; reticulated foams; reticulated thermoplastic films; and thermoplastic scrims. Suitable woven and nonwoven materials may comprise of natural fibers such as wood or cotton fibers; synthetic fibers such as polyester, polypropylene, or polyethylene fibers; or combinations thereof. If the topsheet includes fibers, the fibers may be spunbond, carded, wet-laid, meltblown, hydroentangled, or otherwise processed as is known in the art. One suitable topsheet comprising a web of staple-length polypropylene fibers is manufactured by Veratec, Inc., a Division of International Paper Company, of Walpole, MA under the designation P-8.

Preferred top sheet for use in the present invention are selected from high loft nonwoven topsheets and apertured film topsheet. Apertured film topsheet typically are pervious to bodily exudates, yet non-absorbent, and have a reduced tendency to allow fluids to pass back through and rewet the wearer's skin. Suitable apertured films include those described in U. S. Pat. Nos. 5,628,097, 5,916,661, 6,545,197, 6,107,539, and PCT Patent Publication WO 00/69382 A2.

Further, suitable topsheet materials for depositing solid excretions thereon may include nonwovens having apertures, which are at least in the portions that are aligned with the feces deposition region of the article. Suitable apertured nonwovens are described in more detail in U.S. Patents 6,414,215, 5,342,338, and 5,941,864 and U.S. Patent Publication 2002/017376. In another embodiment of feces handling articles, such topsheets can be combined with feces handling members, e.g., underlying such topsheets, and which are further described in the abovementioned patent documents.

Suitable formed film topsheets are described in U.S. Pat. Nos. 3,929,135, 4,324,246, 4,342,314, 4,463,045, 5,006,394. Other suitable topsheets may be made in accordance with U.S. Pat. Nos. 4,609,518 and 4,629,643. Such formed films are available from The Procter & Gamble Company of Cincinnati, Ohio as "DRI-WEAVE" and from Tredegar Corporation, based in Richmond, VA, as "CLIFF-T."

Preferably, at least a portion of the topsheet is made of a hydrophobic material or is treated to be hydrophobic in order to isolate the wearer's skin from liquids contained in the absorbent core. If the topsheet is made of a hydrophobic material, preferably at least a portion of the upper surface of the topsheet is treated to be hydrophilic so that liquids will transfer through the topsheet more rapidly. The topsheet can be rendered hydrophilic by treating it with a surfactant or by incorporating a surfactant into the topsheet. Suitable methods for treating the topsheet with a surfactant include spraying the topsheet material with the surfactant and/or immersing the material into the surfactant. A more detailed discussion of such a treatment and hydrophilicity is contained in U.S. Pat. Nos. 4,988,344, 4,988,345, and 4,950,254. A more detailed discussion of some suitable methods for incorporating a surfactant in the topsheet 24 can be found in U.S. Statutory Invention Registration No. H1670. Alternatively, the topsheet may include an apertured web or film which is hydrophobic. This may be accomplished by eliminating the hydrophilizing treatment step from the production process and/or applying a hydrophobic treatment to the topsheet, such as a polytetraflouroethylene compound like SCOTCHGUARD or a hydrophobic lotion composition, as described below. In such embodiments, it is preferred that the apertures be large enough to allow the penetration of aqueous fluids like urine without significant resistance.

Any portion of the topsheet may be coated with a lotion as is known in the art. Examples of suitable lotions include those described in U.S. Pat. Nos. 5,607,760, 5,609,587, 5,635,191, 5,643,588, and 5,9680,25. The lotion may function alone or in combination with another agent as the hydrophobizing treatment described above. The topsheet may also include or be treated with antibacterial agents, some examples of which are disclosed in PCT Publication No. WO 95/24173. Further, the topsheet, the outer cover or any portion of the topsheet or outer cover may be embossed and/or matte finished to provide a more cloth like appearance.

The topsheet may comprise one or more apertures to ease penetration of exudates therethrough, such as urine and/or feces (solid, semi-solid, or liquid). The size of at least the primary aperture is important in achieving the desired waste encapsulation performance. If the primary aperture is too small, the waste may not pass through the aperture, either due to poor alignment of the waste source and the aperture location or due to fecal masses having a diameter greater than the aperture. If the aperture is too large, the area of skin that may be contaminated by "rewet" (from the article) is increased. Typically, the aperture should have an area of between about 10 cm² and about 50 cm². The aperture preferably has an area of between about 15 cm² and 35 cm².

Further, the topsheet may be fully or partially elasticated or may be foreshortened so as to provide a void space between the topsheet and the core. Exemplary structures including elasticized or foreshortened topsheets are described in more detail in U.S. Pat. Nos. 4,892,536, 4,990,147, 5,037,416, and 5,269,775. CUFF

The absorbent article further comprises a first cuff 16 along a longitudinal edge 22 of the topsheet 10. This first cuff 16 is useful for providing improved containment of liquids and other body exudates. First cuffs 16 may also be referred to as outer leg cuff, leg bands, side flaps, leg cuffs or elasticized cuffs. U.S. Pat. No. 3,860,003 describes a disposable diaper which provides a contractible leg opening having a side flap and one or more elastic members to provide an elasticized leg cuff.

The first cuff 16 may be constructed in a number of different configurations, including those described in US Pat. Nos. 3,860,003, 4,636,207, 4,695,278, 4,704,115, 4,795,454, 4,900,317, 4,909,803 (Reissued as USRE34920), 5,085,654, 5,492,751, 6,476,288 and SIR H1630.

Additionally, an absorbent article of the present invention may include one or more second cuffs that also provide improved containment of liquids and other body exudates. Second cuffs may also be referred to as barrier leg cuffs, inner leg cuffs or "stand-up" elasticized flaps. U.S. Pat. Nos. 4,808,178 and 4,909,803 (Reissued as USRE34920) describe disposable diapers having "stand-up" elasticized flaps that improve the containment of the leg regions.

First cuff and second cuff may both be provided by way of a dual cuff, as exampled in U.S. Pat. Nos. 4,695,278 and 4,795,454. Additional cuffs may be provided in an article of the present invention as detailed in US Statutory Invention Registration H1630.

### BACKSHEET

The backsheet 12 may or may not be impervious to fluids (e.g., menses, urine, and/or runny feces). Accordingly, one embodiment of the backsheet is manufactured from a thin plastic film, although other flexible liquid impervious or pervious materials may also be used. As used herein, the term "flexible" refers to materials which are compliant and will readily conform to the general shape and contours of the human body. The backsheet 12 prevents the exudates absorbed and contained in the absorbent core from wetting articles which contact the absorbent article such as bedsheets, pants, pajamas and undergarments. The backsheet 12 may thus comprise a woven or nonwoven material, polymeric films such as thermoplastic films of polyethylene or polypropylene, and/or composite materials such as a film-coated nonwoven material (i.e., having an inner film layer and an outer nonwoven layer). A suitable backsheet 12 is a polyethylene film having a thickness of from about 0.012 mm (0.5 mils) to about 0.051 mm (2.0 mils). Exemplary polyethylene films are manufactured by Clopay Corporation of Cincinnati, Ohio, under the designation BR-120 and BR-121 and by Tredegar Film Products of Terre Haute, Ind., under the designation XP-39385. The backsheet 12 is preferably embossed and/or matte-finished to provide a more clothlike appearance. Further, the backsheet 12 may permit vapors to escape from the absorbent core (i.e., the backsheet is breathable) while still preventing exudates from passing through the backsheet. The size of the backsheet 12 is dictated by the size of the absorbent core 18 and the exact absorbent article design selected.

The backsheet 12 and the topsheet 10 are positioned adjacent a garment facing surface and a wearing facing surface, respectively, of the absorbent core. The absorbent core 18 is preferably joined with the topsheet 10, the backsheet 12, or both in any manner as is known by attachment means such as those well known in the art. However, embodiments of the present invention are envisioned wherein portions of the entire absorbent core are unattached to one or both of the topsheet 10 and the backsheet 12.

For example, the backsheet 12 and/or the topsheet 10 may be secured to the absorbent core 18 or to each other by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minn. under the designation HL-1258 or H-2031. The attachment means will preferably comprise an open pattern network of filaments of adhesive as is disclosed in US Pat. No. 4573986. An exemplary attachment means of an open pattern network of filaments comprises several lines of adhesive filaments swirled into a spiral pattern such as illustrated by the apparatus and method shown in US Patents 3,911,173, 4,785,996 and 4,842,666. Alternatively, the attachment means may comprise heat bonds, pressure bonds, ultrasonic bonds, dynamic mechanical bonds, or any other suitable attachment means or combinations of these attachment means as are known in the art.

The backsheet 12b preferably includes an inner and outer layer, each of which can be bonded to the other by a variety of means known in the art, including thermal bonds, adhesive bonds, ultrasonic lamination, or the like. Intimate bonding of the inner and outer layers in the graphic regions reduces light diffraction and thus improves the brightness and overall visibility of the graphic. Adhesive bonding can also be accomplished using adhesive slot coating, high frequency oscillation patterns, for example in swirl or spray patterns, and other fine denier and/or high coverage application techniques. Suitable laminate adhesives, which can be applied continuously or intermittently, can be obtained from Findley Adhesives, Inc. or from National Starch and Chemical Company.

The outer layer (or outer cover) of the backsheet can be made in a variety of forms using different processes. For example, the outer layer may be formed as a carded web, a bonded carded web, a spunbond web, a needled fabric, a woven fabric, or the like to provide a generally cloth-like texture to the wearer. Other additives such as titanium dioxide can represent about 0.5% or less, particularly about 0.3% or less, of the outer layer. In one particular embodiment, the outer layer comprises a spunbond web formed of about 99.5 to 100% polypropylene resin and about 0.5% or less other additives. The outer layer is desirably a lightweight material having a basis weight of about 15 to about 30 gsm and more preferably from about 15 to about 25 gsm.

### ABSORBENT CORE

The articles of the present invention additionally comprise one or more absorbent cores 18. The absorbent core 18 is at least partially disposed between the topsheet 10 and the backsheet 12 and may take on any size or shape that is compatible with the disposable absorbent article. Exemplary absorbent structures for use as the absorbent core of the present invention that have achieved wide acceptance and commercial success are described in US Patents 4,610,678, 4,673,402, and 4,888,231, and 4,834,735. The absorbent core may further comprise the dual core system containing an acquisition/distribution core of chemically stiffened fibers positioned over an absorbent storage core as detailed in U.S. Patents 5,234,423 and 5,147,345.

In general, the absorbent core 18 is capable of absorbing or retaining liquids (e.g., menses, urine, and/or other body exudates). The absorbent core 18 is preferably compressible, conformable, and non-irritating to the wearer's skin. The absorbent core 18 may be manufactured in a wide variety of sizes and shapes (e.g., rectangular, oval, hourglass, "T" shaped, dog bone, asymmetric, etc.). The absorbent core 18 may include any of a wide variety of liquid-absorbent materials commonly used in absorbent articles, such as comminuted wood pulp, which is generally referred to as airfelt. Examples of other suitable absorbent materials for use in the absorbent core include creped cellulose wadding; meltblown polymers including conform, chemically stiffened, modified or cross-linked cellulosic fibers; synthetic fibers such as crimped polyester fibers, peat moss; tissue including tissue wraps and tissue laminates; absorbent foams; absorbent sponges, superabsorbent polymers; absorbent gelling materials; or any equivalent material or combinations of materials, or mixtures of these.

As discussed herein "absorbent gelling materials" and "superabsorbent polymers" are those materials that. upon contact with aqueous fluids, such as bodily fluids, imbibes such fluids and form hydrogels. These absorbent gelling materials are typically capable of absorbing large quantities of aqueous bodily fluids, and further capable of retaining such absorbed fluids under moderate pressures. These absorbent gelling materials are typically in the form of discrete, nonfibrous particles. Other forms, such as fibers, foams, sheets, strips, or other macrostructures, are also suitable for use herein. Suitable absorbent gelling materials in the form of open cell foams may include those disclosed in U.S. Pat. Nos. 3,563,243, 4,554,297, 4,740,520, and 5,260,345.

The configuration and construction of the absorbent core 18 may also be varied (e.g., the absorbent core may have varying caliper zones and/or have a profile so as to be thicker in the center; hydrophilic gradients; superabsorbent gradients; or lower average density and lower average basis weight zones, e.g., acquisition zones; or may comprise one or more layers or structures). The total absorbent capacity of the absorbent core 18 should, however, be compatible with the design loading and the intended use of the absorbent article. Further, the size and absorbent capacity of the absorbent core may be varied to accommodate different uses such as diapers, incontinence pads, pantiliners, regular sanitary napkins, and overnight sanitary napkins, and to accommodate wearers ranging from infants to adults. The absorbent core 18 can include other absorbent components that are often used in absorbent articles, for example, a dusting layer, a wicking or acquisition layer, or a secondary topsheet for increasing the wearer's comfort.

In certain embodiments of the present invention, the absorbent article may also include a sublayer disposed between the topsheet 10 and the backsheet 12. The sublayer may be any material or structure capable of accepting, storing or immobilizing bodily exudates. Thus, the sublayer may include a single material or a number of materials operatively associated with each other. Further, the sublayer may be integral with another element of the absorbent article or may be one or more separate elements joined directly or indirectly with one or more elements of the article. Further, the sublayer may include a structure that is separate from the core or may include or be part of at least a portion of the core.

Suitable materials for use as the sublayer may include large cell open foams, macro-porous compression resistant nonwoven highlofts, large size particulate forms of open and closed cell foams (macro and/or microporous), highloft nonwovens, polyolefin, polystyrene, polyurethane foams or particles, structures comprising a multiplicity of vertically oriented looped strands of fibers, absorbent core structures described above having punched holes or depressions, and the like. One embodiment of a sublayer includes a mechanical fastening loop landing element, having an uncompressed thickness of about 1.5 millimeters available as XPL-7124 from the 3M Corporation of Minneapolis, Minnesota. Another embodiment includes a 6 denier, crimped and resin-bonded nonwoven highloft having a basis weight of 110 grams per square meter and an uncompressed thickness of 7.9 millimeters which is available from the Glit Company of Wrens, Georgia. Other suitable absorbent and nonabsorbent sublayers are described in U.S. Patent Nos. 6,680,422 and 5,941,864. Further, the sublayer, or any portion thereof, may include or be coated with a lotion or other known substances to add, enhance or change the performance or other characteristics of the element.

### GRAPHIC

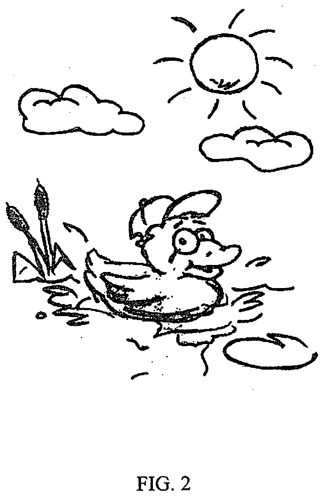

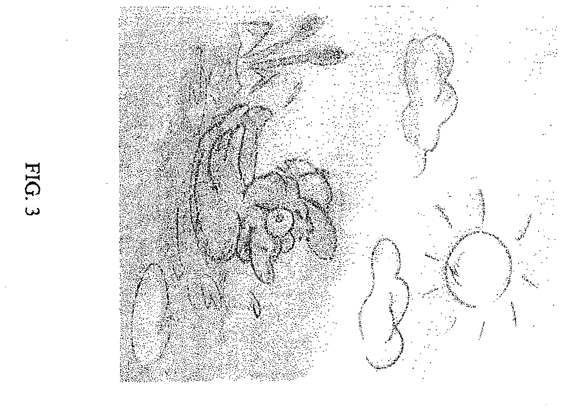

The disposable absorbent articles of the present invention further comprises a graphic 14 that is printed on a garment-facing surface of the backsheet wherein the graphic 14 further comprises a photochromic ink composition that becomes visible to the naked human eye upon exposure to ultraviolet light. Figure 2 shows the graphic 14 of Figure 1 prior to exposure to ultraviolet radiation while Figure 3 shows the same graphic 14 after being exposed to ultraviolet radiation. The graphic 14 of Figure 2 takes on various colors other than the original color of the backsheet on which it is applied. According to the present invention, the graphic comprises the photochromic ink composition which is solvent based and this composition comprises up to about 0.1%, 0.25%, 0.5%, 0.75%, or 1%, by weight, of a dye.

Suitable dyes include but are not limited to spiro (2H-2,3-(3H)naphtha(2,1-b)(1,4)oxazine-1,3-dihydro-1,3,3-trimethyl-6'-(1-piperidinyl) (commercially available as photo chromic violet, CAS No. 114747454), (1,3-dihydro-1,3,3-trimethyl-6'(2.3-dihydro-1H-indol-1-yl)spiro(2H-indole-2,8'-(3H)naphtha (2,1-b)(1,4) oxazine) (commercially available as photo chromic blue, CAS No. 120-12-7), and 3,3-diphenyl-3H-naphtho(2,1-b) pyran (commercially available as photo chromic yellow, CAS No. 4222-20-2). Some photochromic ink compositions that are suitable for use in the present invention include Colorsine Kavi Blue, Colorsine Kirin Purple, Colorsine Kurry Yellow, Colorsine Krisp Red, which are all commercially available from United Polymer Technologies, Inc.

These solvent based photochromic ink compositions further include from about 20%, 22%, or 25% to about 35%, 38%, 40%, or 43%, by weight of the solvent, of a resin or wax. The solvent is present in these solvent based photochromic inks in an amount of from about 57%, 60%, 62%, or 65% to about 75% or 80%. Suitable solvents include alcohols, acetates, and combinations thereof. In particular, suitable alcohols include, but are not limited to, iso-propyl alcohol, n-propyl alcohol, ethanol, methanol, and combinations thereof. Likewise, suitable acetate solvents include, but are not limited to, isopropyl acetate, n-propyl acetate, and combinations thereof. On the other hand, where the photochromic ink is water based, the ink comprises from about 2%, 3%, or 5% to abut 6%, 8%, or 10%, of a dye. These water based photochromic ink compositions comprise from about 30% or 33% to about 37% or about 40% of a resin or wax. Finally, the water based photochromic ink comprises from about 50%, 55%, or 59% to about 60%, 65%, or 68% water.

Suitable photochromic ink compositions may be selected from what are generally known as class A, B, and C materials, and even combinations thereof. Class A encompasses spiro-indolino-naphthoxazines as disclosed in U.S. patents 3,562,172, 4,342,668, 4,440,672 and 4,699,473. In these compounds, basically the same photocoloration reaction is involved as in the photochromies of class C. Class A further comprises spirobenzopyrans as described in Japanese patent publication No. 61-1004, published January 13, 1986, and spiro-benzo(or naphto)thiopyrans. Class B comprises fulgides and fulgimides, disclosed for example in U.S. patent No. 4,172,629, 4,145,536. 4,220,708 and U.K. patents No. 1,442,628 and 2,002,752. Fulgides are derivatives of bis-methylene succinic anhydride, and fulgimides are derivatives of bis-methylene succinic imide where the imide nitrogen may be substituted by alkyl, aryl or aralkyl. Class C comprises spiro(1,8a)-dihydroindolizines, disclosed and described for example in German patent publications No. DE-A-29 06 193, 32 20 257, 33 20 077 and 35 21 432. The photochromic reaction involves the opening of a heterocyclic ring at the spiro location to form a betain-like ionic structure comprising an oxygen anion and a nitrogen cation. Additional suitable photochromic ink compositions are detailed US Patent 5,807,625 and 5,630,869, both to Amon et al. and assigned to Sicpa Holding, S.A.

Generally, the graphic 14 shall comprise at least the photochromic ink which forms an image that is visible upon exposure to ultraviolet light, e.g., sunlight. In other embodiments. The graphic further comprises (i) a permanent ink that is present prior to and after exposure of the article to UV light, and/or (ii) a permanent appearing ink composition, and/or (iii) a disappearing ink composition. For example, the graphic 14 may additionally comprise permanent ink compositions that also are incorporated to form one or more images in conjunction with the photochromic ink composition. Additionally, another embodiment may comprise a permanent appearing ink composition as part of the graphic 14 such that the graphic changes via the appearance of a new color upon when this permanent appearing ink composition is contacted with fluid like water or urine. For instance, this permanent appearing ink composition either appears upon wetting or changes color upon wetting and the appearing color is then permanent. Another option for an additional ink type for the graphic is a disappearing ink composition that becomes invisible upon insult with a liquid. Any of these additional ink compositions may be used one with another in combination with the photochromic ink composition.

According to the present invention, a disappearing ink composition may comprise from about 1% to about 10%, by weight of the composition, of a pigment. More preferably, the composition comprises from about 2% to about 8% and most preferably from about 3% to about 7% of the pigment.

The solid pigments particles used in the present invention include, but not limited to examples, pigment Yellow (C.I. 14), pigment Red (C.I. 48:3), pigment Blue (C.I. 15:4), pigment Black (C.I. 7), and combinations thereof.

Suitable dyes include water soluble ink colorants like direct dyes, acid dyes, base dyes, and various solvent soluble dyes. Examples include, but are not limited to, FD&C Blue 1 (C.I. 42090:2), D&C Red 6(C.I. 15850), D&C Red 7(C.I. 15850:1), D&C Red 9(C.I. 15585:1), D&C Red 21(C.I. 45380:2), D&C Red 22(C.I. 45380:3), D&C Red 27(C.I. 45410:1), D&C Red 28(C.I. 45410:2), D&C Red 30(C.I. 73360), D&C Red 33(C.I. 17200), D&C Red 34(C.I. 15880:1), and FD&C Yellow 5(C.I. 19140:1), FD&C Yellow 6(C.I. 15985:1), FD&C Yellow 10(C.I. 47005:1), D&C Orange 5(C.I. 45370:2), and combinations thereof.

Without being limited by theory, the photochromic ink composition comprises photochromic crystals that upon exposure to UV light undergo a temporary chemical change and are nearly broken apart rendering the change in color that is perceived. When the UV light is removed the molecules of the ink (which is initially colorless) return to their original bonded structure as depicted below which renders a colored ink.

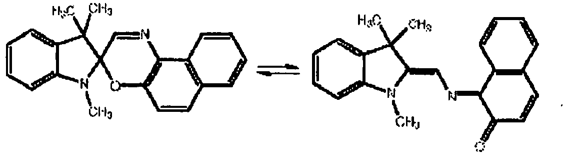

Suitable solvents for the photochromic ink compositions as well as the additional ink compositions described herein are water-based or solvent-based inks. Solvents may be selected from the group consisting of water, toluene, methyl alcohol, ethyl alcohol, and combinations thereof. The photochromic ink composition may comprise from about 40%, 50%, or 60% to about 70%, 80%, or 90%, by weight of the composition, of the solvent.

The photochromic ink composition of the present invention may exhibit an Optical Density of from about 0.10, 0.20, 0.30 to about 0.40, 0.50, 0.70, 0.80, or 1.00.

Alternatively, the photochromic ink composition may exhibit an IODF10, which is the percentage loss in initial Optical Density 10 seconds after removing a UV source, like sunlight. In certain embodiments, the ink composition has an IODF10 value of from about 0.05, 0.07, 0.08, or 0.10 to about 0.30, 0.40, 0.50, 0.60, 0.70, 0.80, or 0.90.

Absorbent articles suitable for use as the present invention include diapers, training pants, incontinence products, diaper pants, disposable underwear, or the like. Suitable training pants and diaper pants can have seamed side portions or refastenable side portions. The present invention is particularly suited for use with training pants or diaper pants to aid in toilet training. Particular diapers and training pants suitable for use with the present invention are disclosed in U.S. Patent Nos. 3,860,003, 4,636,207, 4,695,278, 4,704,115, 4,795,454, 4,900,317, 4,909,803 (Reissued as USRE34920), 5,085,654, 5,492,751, 6,476,288, 6,627,787, 5,507,760, 5,609,587, 5,635,191, 5,643,588, 6,118,041, SIR H1630, 5,246,433, 5,769,838, 5,899,895, 5,899,896, and 6,120,487. Additional patents discussing suitable training pants are disclosed earlier herein.

The article of the present invention may also comprise an elastic waist feature that provides improved fit and containment; and a fastening system which forms a side closure which maintains the first waist region and the second waist region in an overlapping configuration such that lateral tensions are maintained around the circumference of the absorbent article to maintain the absorbent article on the wearer. The absorbent article may also comprise elasticized side panels (not shown) in the waist regions and to provide an elastically extensible feature that provides a more comfortable and contouring fit and more effective application of the absorbent article. In certain embodiments, the elasticized side panels are positioned such that a front and rear side panel are joined to one another along their longitudinal edges. This joining along the longitudinal edges of the panels may be permanent or refastenable. For permanent joints, the panels may be adhered to one another via ultrasonic bonding, high tack, adhesives, etc. For refastenable joints, the panels may be joined via hook and loop fasters, mild co-adhesive materials, low tack adhesives, etc.

Another component that may be included in the articles or composites of the present invention is a hydrophobic surface coating as disclosed in copending US application Serial No. 11/055,743 (P&G Case 9528M, filed on February 10, 2005 in the name of K. Catalan). This hydrophobic surface coating may be paired with the barrier coating of the present invention on the barrier layer or may be disposed adjacent to one or more additional components of the absorbent articles or composites of the present invention. For instance, this hydrophobic surface coating may be disposed on an interior surface of one or more leg cuffs, waist portions, or other areas of the absorbent article.

### OPTICAL DENSITY MEASUREMENT METHOD

In order to determine the value of the Optical Density and the IODF10, the following steps should be followed.
1. Select a sample area of the printed nonwoven for analysis.
2. Place 1 ply of the sample over a PG2000 white standard board such as is commercially available from Sun Chemical's Vivitek Division, Charlotte, NC.
3. It should be noted that the area to be measured should be at least about 4.5 mm in diameter to measure across the width of the SpectroEye (commercially available from the Gretag MacBeth Company or X-Rite, Inc. of Grandville, MI.
4. The aperture of the SpectroEye should be placed over the sample and it should be confirmed that only sample material can be viewed within the instrument's aperture.
5. Expose the sample to a UV light source (e.g., lamp (40W fluorescent lamp or 5-6 kW incandescent light bulb or even sunlight) for at least one minute or until the photochromic image has been properly exposed and is at a steady state Optical Density.
6. Immediately, toggle through the measurement menu to select the "Read and Record" option for the color (L*, a*, and b*) and Optical Density values for each sample.
7. Measure the sample in three separate locations for each color of interest contained within the sample.
8. For each color (i.e., ink), measure and record three color and density readings.
9. For each set of measurements, use the three recordings to calculate and report the average and a standard deviation.
10. For each sample, the chroma, hue, and total color differences may be calculated using the test sample and reference L*, a*, and b* values. Note - the software of the SpectroEye may be configured to perform these calculations.
11. The L*, a*, and b* values are reported to the nearest 0.1 while the density is reported to the nearest 0.01.
12. The Optical Density shall equal the initial density measurement while the IODF10 shall equal the Optical Density 10 seconds after the UV light source is removed from the sample.

### METHOD OF PRINTING GRAPHIC

The articles of the present invention are printed with the graphic comprising the photochromic ink composition using ink jet printers, flexographic printing presses, gravure printing presses, or a combination thereof. The graphic may be printed on a number of article components including, but not limited to, the backsheet, topsheet, cuffs, etc. It is more likely, however, that the graphic is printed on a surface that will be subject to sun exposure during wear of the article.

### EXAMPLES

An absorbent article which is a diaper, training pant, or adult incontinence product is made as detailed in any one of US Patents 3,860,003, 4,636,207, 4,695,278, 4,704,115, 4,795,454, 4,900,317, 4,909,803 (Reissued as USRE34920), 5,085,654, 5,492,751, 6,476,288, 6,627,787, 5,507,760, 5,609,587, 5,635,191, 5,643,588, 6,118,041, SIR H1630, 5,246,433, 5,769,838, 5,899,895, 5,899,896, and 6,120,487. The backsheet of the disclosed absorbent articles is printed with a graphic of an anthropomorphous character (a cartoon duck in a pond with a sun shining overhead) on its garment facing surface as shown in Figure 2 depicts the character of Figure 1 after the article has been exposed to sunlight. The graphic comprises one or more of the photochromic ink compositions detailed below.

### Examples 1 - 3

### Part i

3 parts of a photochromic compound of class A above are first dissolved in 57 parts of cellulose acetate propionate (CAP 504 - 0,2 - Eastman), 20 parts of toluene and 20 parts of ethanol by heating the mixture to 60 degrees C for 15 minutes. The mixture is then dried at 80 degrees C under reduced pressure until total evaporation of solvents. The solid solution obtained is broken up and then micronized, and the finely divided particles obtained having a size of 1 to 20 microns are incorporated in the following ink formulation by means of a ball mill.

### Part 2

An aqueous heliographic and flexographic ink is formulated as follows:
48% solids emulsion of acrylic polymers in water (Joncryl 89 - Johnson)
49% solids emulsion of acrylic polymers in water (Joncryl 74 - Johnson)
Antifoaming agent (Silicone DC 3 - Dow Coming)
Polyethylene wax (Ceridust VP 3715 - Hoechst)
Isopropyl alcohol
Water

A solid solution of photochromic dye in cellulose acetate proprionate, are prepared as detailed above. The two emulsions of acrylic polymers are mixed with the antifoaming agent. Polyethylene wax is dispersed therein for ten minutes in a ball mill. Finally, isopropyl alcohol, water and the photochromic dyestuff are incorporated into the mixture by means of a ball mill. After conventionally printing the backsheet and drying, the colorless print turns blue when irradiated with light having a wavelength of about 366 nm.

### Example 2

The steps of Example 1 are repeated with the exception that a photochromic of Class B above is substituted for the same amount of photochromic A. An aqueous photochromic printing ink for gravure and flexographic printing was obtained. After conventionally printing the backsheet and drying, the colorless print turns pink when irradiated with light having a wavelength of about 366 nm.

### Example 3

The steps of Example 1 are repeated with the exception that a photochromic of Class C above was substituted for the same amount of photochromic A. An aqueous photochromic printing ink for gravure and flexographic printing was obtained. After conventionally printing the backsheet and drying, the colorless print turns green when irradiated with light having a wavelength of about 366 nm.

### Example 4 - Photochromic intaglio ink

Part 1 of Example 1 is repeated to prepare a solid solution of a photochromic of class A in cellulose acetate propionate, having a concentration of 5% weight, and a particle size comprised between 1 and 20 microns. Then, a photochromic intaglio ink is prepared by mixing the following ingredients under gentle heating on a three roller mill:
Addition product of tung oil and maleic acid modified phenolic resin in a high boiling mineral oil (PKWF 28 DEG 31)
1. Long oil alkyd resin in ink solvent 27/29 (Shell Ch. Ind) 7.5 %
2. Alkylphenolic resin modified with raw tung oil in ink solvent 27/29 (Shell Ch. Ind.) 16%
3. Polyethylene wax 1.5 %
4. Solid solution of photodye A in cellulose acetate propionate (Example 1, part 1) 39.7 %
5. Cobalt octoate (11% metal) 0.05 %
6. Magnanese octoate (10% metal) 0.10 %
7. Lead octoate (38% metal) 0.15 %

### Example 5- Photochromic offset and litho prinking ink

Part 1 of Example 1 is repeated to prepare a solid solution of 5% by weight of a photochromic compound of class A above in cellulose acetate propionate having a particle size of 1 to 20 microns. Then, a photochromic offset and litho printing ink is prepared by mixing the following ingredients:
1. Phenolic resin modified rosin cooked with linseed oil 24 %
2. Long oil alkyd (Alftalat AL 810 - Reichhold Albert Chemie AG) <SEP>35 %
3. Polyethylene wax (PE 130 - Hoechst) 2 %
4. Ink solvent 27/29 (Shell Industrial Chemicals)<SEP>2 %
5. Cooked linseed oil<SEP>5 %
6. Solid solution of photodye A in cellulose acetate propionate (5%) (Example 1, part1) 26.2 %
7. Titanium dioxide 5%
8. Cobalt octoate (10% cobalt) 0.8 %

The ink, after printing and drying, gives a blue photochromic coloration under irradiation.

This Example is repeated except that a photochromic of class B and class C, respectively, was replaced for the photochromic of class A. These inks, after printing the backsheet and drying, results in pink or green photochromic colorations, respectively, under irradiation.

### Example 6 - Photochromic intaglio printing ink

### Part 1- microcapsules

Five parts of a photochromic of class A are dissolved in 50 parts of chloroform to obtain a clear solution A. Following the encapsulating procedure described in U.S. patent specification No. 4,517,141, solution A is added slowly at room temperature to 270 parts of a stirred solution of 0.65 parts of polyvinyl alcohol, 1,35 parts of xanthan gum, and 9 parts of diethyl tolylene diamine in 268 parts of distilled water. The shearing rate is increased for 1 minute resulting in the formation of a fine oil-in-water emulsion. Then, the stirrer speed is reduced, and 180 parts of a solution of 20 parts of powdered hexamethylene diisocyanate, sodium bisulfite adduct, in 160 parts of a colloid solution containing 0.25% of polyvinyl alcohol and 0.50% of xanthan gum, are added to the diamine/photochromic emulsion.

Emulsification is enhanced by applying high shear rate conditions, and the slurry obtained is transferred into a reaction vessel equipped with a reflux condenser and heated for 6 hours to about 60 degrees C under gentle stirring. Then, the slurry is spray dried. Dry, transparent microcapsules are obtained having a diameter between 1 and 20 micrometers and containing about 10% of the photochromic. When irradiated with light having a wavelength of about 366 nm, a strong blue photochromic effect is obtained.

### Part 2 - printing ink

An intaglio (steel plate) printing ink is obtained by blending the following ingredients under gentle warming on a three roller mill:
1. Addition product of tung oil and maleic acid modified phenolic resin in a high boiling mineral oil (BKDF 28/31, Haltermann) 35 %
2. Long oil alkyd resin in ink solvent 27/29 (Shell) 7.5 %
3. Raw tung oil modified alkylphenol resin in ink solvent 27/29 (Shell) 16 %
4. Polyethylene wax 1.5 %
5. Microcapsules of part 1 of this Example 35 %
6. Titanium dioxide 4.7 %
7. Cobalt octoate (11% metal) 0.05 %
8. Manganese octoate (10% metal) 0.10 %
9. Lead octoate (38% metal) 0.15 %

After conventionally printing the backsheet and drying, a nearly colorless print is obtained which turns blue when irradiated with light having a wavelength of about 366 nm.

When the photochromic of class A in this Example is replaced in part 1 by the same amount of a photochromic of classes B or C, intaglio printing inks are also obtained.

### Examples 10 - 13 -Photochromic gravure printing ink

### Example 10

A gravure ink, based on a nitrocellulose resin, is prepared from the following ingredients using the indicated quantities:
1. Nitrocellulose (A280 - Société Nationale des Produits Explosifs S.N.P.E., France) 15 %
2. Dibutyl phthalate 6 %
3. Ethanol 34.5 %
4. Ethyoxypropanol 10 %
5. Ethyl acetate 31.5 %
6. Photochromic of class A 3 %

All the ingredients are mixed together at 50 degrees C to obtain a clear solution. The ink is adjusted to the suitable printing viscosity by means of adding ethyl acetate. When the photochromic of class A in this Example is replaced by the same amount of a photochromic of class B or class C, excellent photochromic gravure printing inks are also obtained.

### Example 11

A gravure printing ink, based on a polyamide resin, is prepared from the following ingredients using the indicated quantities;
1. Polyamide (Eurelon 962 - Schering) 30 %
2. Ethanol 45 %
3. Propyleneglycol methyl ether 5 %
4. Ethyl acetate 17 %
5. Photochromic of class A 3 %

The manufacturing process of this gravure ink is the same as in Example 10. When the photochromic of class A in this Example is replaced by the same amount of a photochromic of class B or C, excellent photochromic gravure printing inks are also obtained.

### Example 12

A gravure ink, based on an ethylcellulose resin, is prepared from the following ingredients using the quantities indicated and applying the method of Example 10:
1. Ethyl cellulose (N7 - Hercules) 10 %
2. Propyleneglycol methyl ether 10 %
3. Ethanol 28 %
4. Isopropyl acetate 44 %
5. Dibutyl phthalate 5 %
6. Photochromic of class A 3 %

The final printing viscosity is adjusted by means of adding isopropyl acetate.
When the photochromic of class A in this Example is replaced by the same amount of a photochromic of class B or C, excellent photochromic gravure printing inks are also obtained.

### Examples 14-15 - Photochromic flexographic printing ink

### Example 14

The ink, based on a nitrocellulose resin, is formulated as follows:
1. Nitrocellulose (N7 - Hercules) 20 %
2. Fatty amide (Armid O, Armour) 1.5 %
3. Promotex PE 80 A 8 %
4. Dibutyl phthalate 5 %
5. Propyleneglycol methyl ether 18 %
6. Ethanol 35.5 %
7. Ethyl acetate 9 %
8. Photochromic of class A 3 %

All the ingredients are mixed together at 50 degrees C to obtain a clear solution. The final printing viscosity is obtained by adding a suitable quantity of ethyl acetate. When the photochromic of class A in this Example is replaced by the same amount of a photochromic of class B or C, excellent flexographic printing inks are also obtained.

### Example 15

This ink, based on a polyamide resin, is formulated as follows:
1. Polyamide (Eurelon 945 - Schering) 36 %
2. Inalco RL 727 9 %
3. Fractionated gasoline (100-125 degrees C) 27 %
4. Isopropyl alcohol 25 %
5. Photochromic of class A 3 %

The manufacturing process is the same as in Example 12. When the photochromic of class A in this Example is replaced by the same amount of a photochromic of class B or C, excellent flexographic printing inks are also obtained.

## Claims

1. A disposable absorbent article (20) comprising:
a) a liquid pervious topsheet (10);
b) a backsheet (12) that is at least partially joined to the topsheet;
c) an absorbent core (18) disposed at least partially between the topsheet and the backsheet;
d) a first cuff (16) along a longitudinal edge (22) of the topsheet; and
e) a graphic (14) disposed on a surface (24) of said backsheet wherein said graphic comprises a photochromic ink composition that becomes visible to the naked human eye upon exposure to ultraviolet light, wherein said graphic (14) is printed on a garment-facing surface of said backsheet (12), and
wherein said graphic (14) further comprises
(i) a permanent ink that is present prior to and after exposure of the article to ultraviolet light; and/or
(ii) a permanent appearing ink composition; and/or
(iii)a disappearing ink composition.

2. The article (20) of claim 1 wherein said photochromic ink composition exhibits an Optical Density of from 0.10 to 1.00 as measured according to the optical density measurement method disclosed herein, wherein said photochromic ink composition exhibits an IODF10 value of from 0.05 to 0.50 as measured according to the optical density measurement method disclosed herein, wherein said photochromic ink composition is selected from the group consisting of Class A, Class B, Class C, and combinations thereof.

3. The article (20) of any of the preceding claims wherein said article further comprises elasticized side panels, preferably said elasticized side panels are refastenable.

4. The article (20) of any of the preceding claims further comprising at least one second cuff (16) along a longitudinal edge (22) of the topsheet (10), preferably at least one of said cuffs is contacted with a hydrophobic surface coating.

## Patentansprüche

1. Einweg-Absorptionsartikel (20), umfassend:
a) eine flüssigkeitsdurchlässige Oberschicht (10);
b) eine Unterschicht (12), die wenigstens teilweise mit der Oberschicht verbunden ist;
c) einen Absorptionskern (18), der wenigstens teilweise zwischen der Oberschicht und der Unterschicht angeordnet ist;
d) ein erstes Bündchen (16) entlang einem Längsrand (22) der Oberschicht; und
e) eine Grafik (14), die auf einer Oberfläche (24) der Unterschicht angeordnet ist, wobei die Grafik eine photochrome Tintenzusammensetzung umfasst, die bei Aussetzen gegenüber ultraviolettem Licht für das bloße menschliche Auge sichtbar wird, wobei die Grafik (14) auf eine bekleidungsseitige Oberfläche (12) aufgedruckt ist, und
wobei die Grafik (14) ferner umfasst
(i) eine permanente Tinte, die vor und nach dem Aussetzen des Artikels gegenüber ultraviolettem Licht vorhanden ist; und/oder
(ii) eine permanent erscheinende Tintenzusammensetzung; und/oder
(iii) eine verschwindende Tintenzusammensetzung.

2. Artikel (20) nach Anspruch 1, wobei die photochrome Tintenzusammensetzung eine optische Dichte von 0,10 bis 1,00, gemäß Messung nach dem hierin offenbarten Verfahren zur Messung der optischen Dichte, aufweist, wobei die photochrome Tintenzusammensetzung einen IODF10-Wert von 0,05 bis 0,50, gemäß Messung nach dem hierin offenbarten Verfahren zur Messung der optischen Dichte, aufweist, wobei die photochrome Tintenzusammensetzung ausgewählt ist aus der Gruppe bestehend aus Klasse A, Klasse B, Klasse C und Kombinationen davon.

3. Artikel (20) nach einem der vorstehenden Ansprüche, wobei der Artikel ferner elastifizierte Seitenfelder umfasst, vorzugsweise sind die elastifizierten Seitenfelder wiederverschließbar.

4. Artikel (20) nach einem der vorstehenden Ansprüche, ferner umfassend wenigstens ein zweites Bündchen (16) entlang einem Längsrand (22) der Oberschicht (10), vorzugsweise wird wenigstens eines der Bündchen mit einer hydrophoben Oberflächenbeschichtung in Kontakt gebracht.

## Revendications

1. Article absorbant jetable (20) comprenant :
a) une feuille de dessus perméable aux liquides (10) ;
b) une feuille de fond (12) qui est au moins partiellement jointe à la feuille de dessus ;
c) une âme absorbante (18) disposée au moins partiellement entre la feuille de dessus et la feuille de fond ;
d) un premier revers (16) le long d'un bord longitudinal (22) de la feuille de dessus ; et
e) un motif (14) disposé sur une surface (24) de ladite feuille de fond, dans lequel ledit motif comprend une composition d'encre photochromique qui devient visible à l'oeil nu humain lors d'une exposition à une lumière ultraviolette, dans lequel ledit motif (14) est imprimé sur une surface faisant face au vêtement de ladite feuille de fond (12), et
dans lequel ledit motif (14) comprend en outre
(i) une encre permanente qui est présente avant et après exposition de l'article à une lumière ultraviolette ; et/ou
(ii) une composition d'encre permanente qui apparaît ; et/ou
(iii) une composition d'encre qui disparaît.

2. Article (20) selon la revendication 1, dans lequel ladite composition d'encre photochromique présente une densité optique allant de 0,10 à 1,00 telle que mesurée selon le procédé de mesure de densité optique décrit ici, dans lequel ladite composition d'encre photochromique présente une valeur IODF10 allant de 0,05 à 0,50 telle que mesurée selon le procédé de mesure de densité optique décrit ici, dans lequel ladite composition d'encre photochromique est choisie dans le groupe constitué de Classe A, Classe B, Classe C, et des combinaisons de celles-ci.

3. Article (20) selon l'une des revendications précédentes, dans lequel ledit article comprend en outre des pans latéraux élastifiés, de préférence lesdits pans latéraux élastifiés sont repositionnables.

4. Article (20) selon l'une quelconque des revendications précédentes, comprenant en outre au moins un deuxième revers (16) le long d'un bord longitudinal (22) de la feuille de dessus (10), de préférence au moins l'un desdits revers est mis en contact avec un revêtement de surface hydrophobe.
